# EUROPÄISCHE PATENTSCHRIFT

(11) **EP 2 624 764 B1**
(45) Veröffentlichungstag und Bekanntmachung des Hinweises auf die Patenterteilung: **30.12.2015**
(21) Anmeldenummer: 11771066.5
(22) Anmeldetag: 06.10.2011
(51) Int. Cl.: A61B 17/00, C04B 35/48

(54) **SCHNITTSCHABLONE AUS KERAMIK**
CERAMIC CUTTING TEMPLATE
GABARIT DE COUPE EN CÉRAMIQUE

(30) Priorität: 06.10.2010 DE 102010047473
(43) Veröffentlichungstag der Anmeldung: 14.08.2013
(73) Patentinhaber: CeramTec GmbH, 73207 Plochingen (DE)
(72) Erfinder: ESCHLE, Matthias, 72827 Wannweil (DE); PREUSS, Roman, 73230 Kirchheim unter Teck (DE); WECKER, Heinrich, 90542 Eckental (DE)
(74) Vertreter: Uppena, Franz
(86) Internationale Anmeldenummer: PCT/EP2011/067487
(87) Internationale Veröffentlichungsnummer: WO 2012/045826

(56) Entgegenhaltungen:
- EP-A1- 1 514 578
- WO-A1-2009/042110
- WO-A1-2010/112588
- WO-A2-2010/112589
- JP-A- 2006 104 023
- JP-A- 2009 269 812

## Beschreibung

Gegenstand der vorliegenden Erfindung ist eine Schnittschablone bzw. ein Sägeblock, vorzugsweise eine Schnittschablone bzw. ein Sägeblock zum Einsatz in der Medizintechnik.

Schnittschablonen bzw. Sägeblocks werden in der Chirurgie vielfach eingesetzt, um das Operationsfeld vorzubereiten bzw. anzupassen, beispielsweise wenn es darum geht, Implantationen vorzunehmen.

So wird beispielsweise bei jeder Knie-TEP-Implantation eine Schnittschablone bzw. ein Sägeblock auf dem Femur fixiert. Mit dieser Schnittschablone werden im Normalfall drei Schnitte zur Anpassung der Femuroberfläche an die Geometrie der Femurkomponente durchgeführt. Für jeden Schnitt befindet sich in der Schnittschablone eine Führung (3 bzw. 4 Schnittführungen in 1 Schablone). In dieser Führung wird der Schnitt mit einem Schneideinstrument, im Normalfall mit einem oszillierenden Sägeblatt durchgeführt. Die Sägeblätter sowie die Schnittschablonen sind heute grundsätzlich aus biokompatiblen Metalllegierungen gefertigt.

Die Führungsschienen im Sägeblock haben je nach Hersteller eine Breite von 1,2 - 1,5 mm. Bedingt durch das Oszillieren des Sägeblatts und die auftretende Reibung zwischen Sägeblatt und Führungsschiene tritt ein hoher Metallabrieb auf Seiten der Führungsschiene auf. Dieser Abrieb wird nicht bzw. kann nur unzureichend intraoperativ aus der Wunde entfernt werden. Somit kann dieser Abrieb wiederum zur Ursache von Infektionen werden und vor allem zu allergischen Reaktionen des Patienten führen. Aus diesem Grund gilt es, diesen Abrieb grundsätzlich zu reduzieren, insbesondere jedoch, wenn durch den Einsatz einer keramischen Femurkomponente beim potentiellen Allergiker eine Implantatreaktion vermieden werden soll.

Nach heutigem Kenntnisstand entsteht der überwiegende Teil des Metallabriebs durch den Verschleiß der Führungsschienen in der Schnittschablone. Nach ca. 20 - 40-maliger Verwendung einer Schnittschablone im Rahmen von Knie-TEP-Implantationen weisen die Führungsschienen um ca. 0,5 - 1,5 mm vergrößerte Führungsspalte auf. Infolgedessen nimmt die Führungsgenauigkeit der Schnittschablone erheblich ab. Die Folgen für den Chirurgen sind entsprechend, eine präzise Schnittführung des Sägeblattes ist nicht mehr möglich, Ausrichtung und Ebenheit der Schnittflächen des Femurs weisen zunehmend Abweichungen auf. Dies führt zu größeren Spalten zwischen Schnittflächen und Femurkomponente. Diese Spalte müssen intraoperativ durch ein größeres als sonst übliches Volumen an Knochenzement aufgefüllt werden, was einen negativen Einfluss auf die Standzeit des Systems haben kann.

Die der vorliegenden Erfindung zugrunde liegende Aufgabe bestand darin, die Nachteile der Schnittschablonen / der Sägeblöcke des Standes der Technik zu beseitigen und insbesondere:
➢ den Metallabrieb zu reduzieren, wobei eine Reduzierung des Metallabriebs um bis zu 90% gegenüber den bisherigen Metalllösungen angestrebt werden soll;
➢ die Standzeit einer Schnittschablone zu verlängern und damit Kosten einzusparen;
➢ das Allergierisiko sowie das Risiko von Infektionen zu reduzieren.

Die erfindungsgemäße Aufgabe wurde überraschenderweise durch eine Schnittschablone / einen Sägeblock aus Keramik (im Folgenden werden für die erfindungsgemäße Schnittschablone / den erfindungsgemäßen Sägeblock auch die Begriffe Sinterformkörper oder Sinterkörper verwendet) mit den Merkmalen der unabhängigen Ansprüche gelöst. Vorzugsweise Ausgestaltungen finden sich in den Unteransprüchen. Es wurde überraschenderweise festgestellt, dass die Lösung der anstehenden Aufgaben Sinterformkörper mit ganz speziellen Zusammensetzungen erfordert.

Sinterformkörper, die die Lösung der anstehenden Aufgaben ermöglichen, sind sogenannte "yttria-stabilized tetragonal zirconia-Keramiken" auch Y-TZP-Keramiken genannt. Erfindungsgemäß geeignet sind solche Y-TZP-Keramiken, die der Norm für medizinische Anwendungen entsprechen.

### Geeignete Y-TZP-Keramiken enthalten

3 bis 8 Gew.-% Y₂O₃, vorzugsweise 4 bis 6 Gew.-% Y₂O₃, besonders bevorzugt 4,5 bis 5,5 Gew.-% Y₂O₃,
0 bis 0,5 Gew.-% Al₂O₃, vorzugsweise 0,05 bis 0,4 Gew.-% Al₂O₃, besonders bevorzugt 0,1 bis 0,3 Gew.-% Al₂O₃ und
Rest auf 100 Gew.-% ZrO₂, wobei bis zu 3 Gew.-% HfO₂, vorzugsweise bis zu 2 Gew.-% HfO₂ im Zirkonoxid enthalten sein können.

Der Monoklinanteil im ZrO₂ liegt dabei bei weniger als 2 Vol-%, vorzugsweise bei weniger als 1 Vol-%

Die Eigenschaften der geeigneten Y-TZP-Keramiken weisen eine Festigkeit von 900 bis 1600 MPa, vorzugsweise von 1000 bis 1500 MPa auf. Die Korngröße der Keramik bewegt sich dabei in einem Bereich von < 0,5 µm, vorzugsweise in einem Bereich von 0,1 bis 0,3 µm.

Vorzugsweise Ausgestaltungen sind in der folgenden Tabelle aufgeführt:

| | **Beispiel 1** | **Beispiel 2** | |
|---|---|---|---|
| | Wert | Wert | Einheit |
| Yttriumoxid (Y₂O₃) | 4,9 | 5,2 | Gew.-% |
| Hafniumoxid (HfO₂) | 2,0 | 2,0 | Gew.-% |
| Aluminiumoxid (Al₂O₃) | 0,1 | 0,1 | Gew.-% |
| Verunreinigungen | < 0,1 | < 0,1 | Gew.-% |
| Zirkonoxid (ZrO₂) | Rest auf 100 | Rest auf 100 | Gew.-% |

Die Eigenschaften der vorzugsweise eingesetzten Keramiken sind in den folgenden Tabellen aufgeführt.

### Beispiel 1:

| | | | |
|---|---|---|---|
| Korngröße | 0,15 - 0,2 | µm | |
| Farbe | Elfenbein | | |
| Dichte | 6,08 | g/cm³ | DIN EN 623-2 |
| Wasseraufnahmevermögen | 0 | % | ASTM C 373 |
| Festigkeit (4-Punkt-Biegung) | 1400 | MPa | DIN ENV 843-1 |
| Weibull-Modul | 10 | | DIN ENV 843-5 |
| Bruchzähigkeit | 7,5 | MPa√m | ISO 23 146 |
| Vickers-Härte (HV 0,5) | 13 | GPa | DIN ENV 843-4 |
| E-Modul | 210 | GPa | DIN ENV 843-2 |
| Poisson-Zahl | 0,3 | | DIN ENV 843-2 |
| | | | |
| Wärmeausdehnungskoeffizient | | | DIN EN 821-1 |
| 20 - 200 °C | 10,4 | 10⁻⁶ K⁻¹ | |
| 20 - 1000 °C | 11,4 | 10⁻⁶ K⁻¹ | |
| | | | |
| Spezifische Wärmekapazität (20 °C) | 0,4 | kJ / kg K | DIN EN 821-3 |
| Wärmeleitfähigkeit (20 °C) | 2,5 | W/mK | DIN EN 821-2 |

### Beispiel 2:

| | | | |
|---|---|---|---|
| Korngröße | < 0,5 | µm | |
| Farbe | weiß | | |
| Dichte | 6,05 | g/cm³ | DIN EN 623-2 |
| Wasseraufnahmevermögen | 0 | % | ASTM C 373 |
| Gasdurchlässigkeit | 0 | % | |
| Festigkeit (4-Punkt-Biegung) | 1050 | MPa | DIN ENV 843-1 |
| Druckfestigkeit | 2200 | MPa | DIN 51067 T1 |
| Weibull-Modul | >10 | | DIN ENV 843-5 |
| Vickers-Härte (HV 1) | 1250 | | DIN ENV 843-4 |
| E-Modul (dynamisch) | 210 | GPa | DIN ENV 843-2 |
| Poisson-Zahl | 0,3 | | DIN ENV 843-2 |
| | | | |
| Wärmeausdehnungskoeffizient | | | DIN EN 821-1 |
| 20 - 200 °C | 11,1 | 10⁻⁶ K⁻¹ | |
| 20 - 1000 °C | 11,7 | 10⁻⁶ K⁻¹ | |
| | | | |
| Spezifische Wärmekapazität (20 °C) | 0,4 | kJ / kg K | DIN EN 821-3 |
| Wärmeleitfähigkeit (20 °C) | 2,5 | W/mK | DIN EN 821-2 |
| Wärmespannungsparameter R1 | 321 | K | |
| Dielektrizitätskonstante | 29 (1 MHz) | | IEC 672-1 |
| Dielektrischer Verlustfaktor | 0,002 (1 GHz) | | IEC 672-1 |

Bei den erfindungsgemäß aus Y-TZP-Keramiken hergestellten Schnittschablonen bzw. Sägeblöcken ist der Metallabrieb um bis zu 90% gegenüber den bisherigen Schnittschablonen bzw. Sägeblöcken aus Metall reduziert. Die Standzeit der erfindungsgemäßen Schnittschablonen bzw. der erfindungsgemäßen Sägeblöcke im Einsatz ist deutlich verlängert, da nur geringer Verschleiß der Schnittschablonen auftritt. Dies reduziert die Kosten. Außerdem sind das Allergierisiko bzw. die allergischen Reaktionen von Patienten sowie das Risiko von Infektionen reduziert.

Bevorzugt werden die erfindungsgemäßen Schnittschablonen in der Medizintechnik, insbesondere bei Operationen zur Bearbeitung eines Knochens, in bevorzugter Weise bei Knie-TEP-Implantationen verwendet.

Die Vorteile der erfindungsgemäßen keramischen Schnittschablone bzw. die der Keramik, aus der sie hergestellt ist, sind:
➢ Die Schnittschablone weist einen extrem geringen Abrieb auf.
➢ Das Material ist biokompatibel.
➢ Wenn die erfindungsgemäße Schnittschablone mit einem Laser beschriftet wird, ist diese sehr gut sichtbar und lesbar und kann somit Fehlhandhabungen beim Einsatz der Schnittschablone reduzieren.
➢ Die Schnittschablone besitzt gute tribologische Eigenschaften.

Die Herstellung der Schnittschablone erfolgt mittels konventioneller Keramiktechnologie.

Die wesentlichen Prozessschritte sind:
a) Pulvermischung gemäß vorgegebener Zusammensetzung in Wasser ansetzen, Verwendung von Verflüssigern zur Vermeidung der Sedimentation.
b) Homogenisieren im Dissolver (schnelllaufender Rührer).
c) Mahlen in Rührwerkskugelmühle, dabei Erhöhung der spezifischen Oberfläche der Pulvermischung (= Zerkleinerung).
d) Zugabe von organischen Bindern.
e) Sprühtrocknen, dabei entsteht ein rieselfähiges Granulat mit definierten Eigenschaften.
f) Befeuchten des Granulats mit Wasser.
g) Axial oder isostatisch pressen.
h) Spanabhebende Grünbearbeitung, dabei wird unter Berücksichtigung der Sinterschwindung weitgehend die Endkontur abgebildet.
i) Vorbrand, dabei Schwindung auf ca. 98% der theoretischen Dichte. Die noch verbleibenden Restporen sind nach außen geschlossen.
j) Heißisostatisches Pressen unter hoher Temperatur und hohem Gasdruck, dadurch praktisch vollständige Endverdichtung.
k) So genannter Weißbrand, dadurch wird das beim heißisostatischen Pressen erzeugte Ungleichgewicht der Sauerstoffionen in der Keramik ausgeglichen.
l) Hartbearbeitung durch Schleifen und Polieren.
m) Tempern.

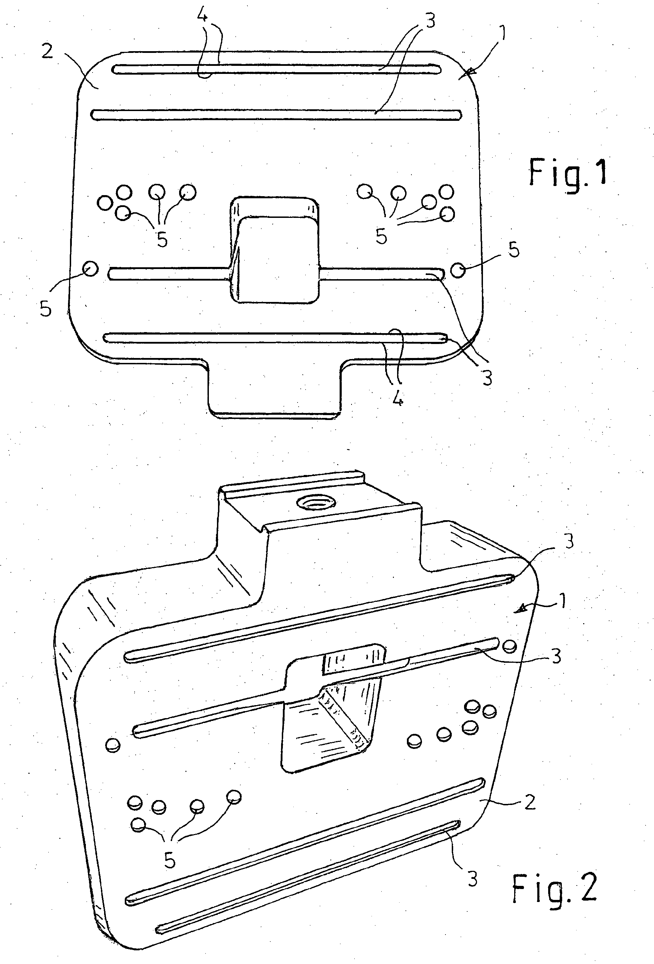

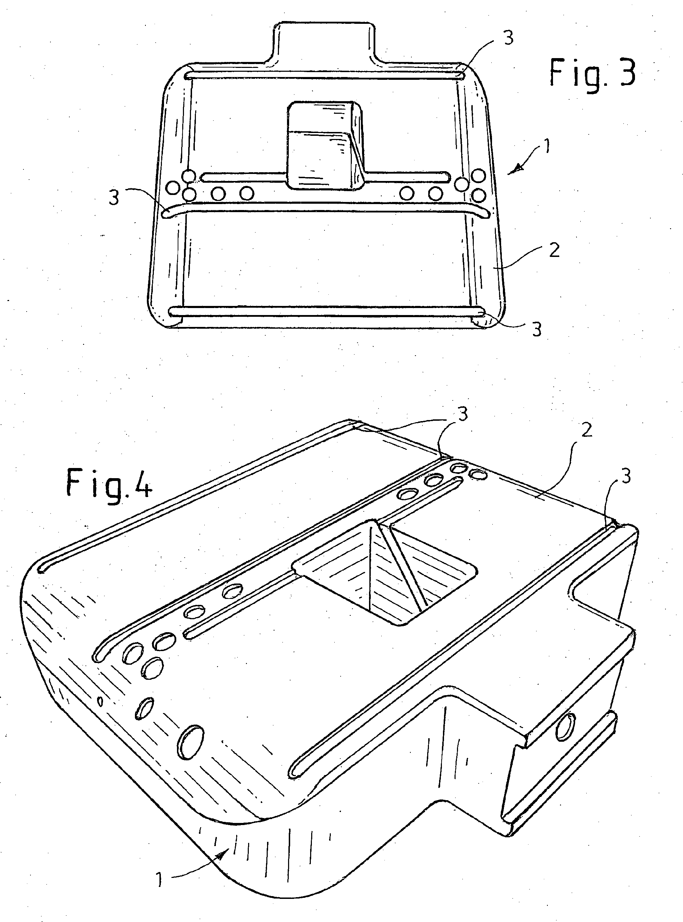

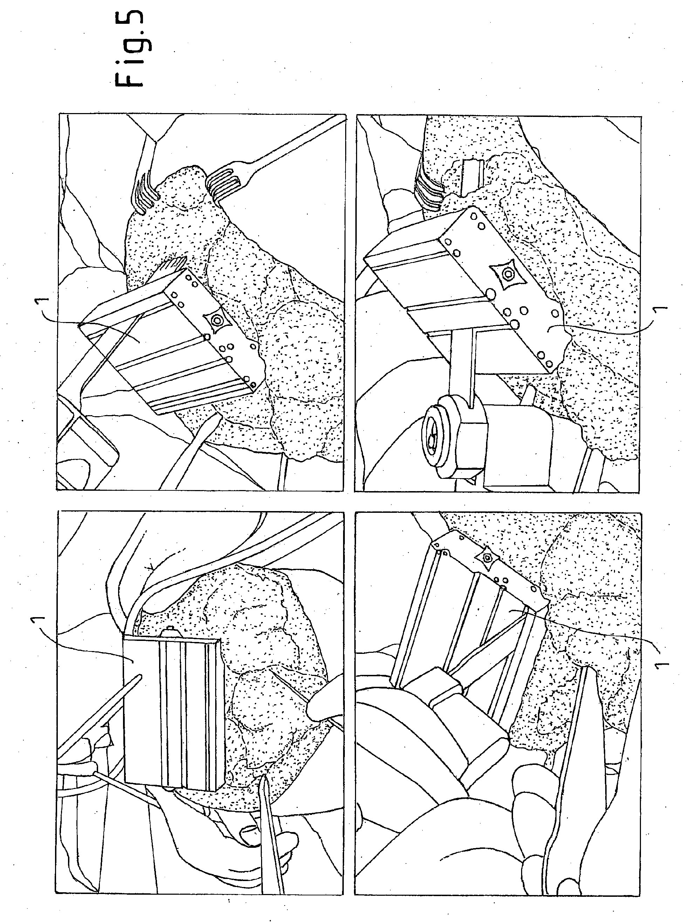

Die Figuren 1 bis 4 zeigen beispielhaft eine erfindungsgemäße Schnittschablone 1 aus Keramik für den Einsatz bei der Implantation eines künstlichen Kniegelenks in verschiedenen Ansichten. Eine derartige Schnittschablone 1 dient zur Führung eines chirurgischen Schneideinstruments, beispielsweise eines Sägeblatts oder eines Bohrers. Figur 5 soll die intraoperative Verwendung einer erfindungsgemäßen Schnittschablone bei der Implantation eines künstlichen Kniegelenks illustrieren.

Die gezeigte Schnittschablone besteht aus einem Grundkörper 2, welcher mit schlitzartigen Ausnehmungen 3 zur Durchführung und passgenauen Führung eines plattenförmigen Schneideinstruments, beispielsweise eines Sägeblatts, versehen ist, wobei die schlitzartigen Ausnehmungen 3 einander gegenüberliegende Führungsflächen 4 aufweist. An diesen Führungsflächen 4 liegt das Schneideinstrument (siehe Figur 5) beim Schnittvorgang an. In den Grundkörper 2 sind Durchgangsbohrungen 5 eingebracht, die zur Verschraubung der Schnittschablone 1 auf dem Femur dienen.

Für den Fachmann ist es selbstverständlich, dass die erfindungsgemäße Schnittschablone je nach Einsatzzweck angepasst werden kann. So kann sie beispielsweise auch in der Form einer Bohrlehre ausgestaltet sein, bei der beispielsweise eine oder mehrere der Ausnehmungen 3 als Durchgangsbohrung zur Durchführung und passgenauen Führung eines Bohrers als Schneideinstrument ausgeführt sind.

Im Rahmen der vorliegenden Erfindung bezeichnen die Begriffe Sinterformkörper / Sinterkörper eine Keramik in Form von bzw. zur Verwendung als Schnittschablone bzw. Sägeblock. Als Schnittschablone bzw. Sägeblock im Sinne der vorliegenden Erfindung soll auch eine erfindungsgemäß ausgestaltete Bohrlehre verstanden werden.

Aus dem Vorstehenden ergibt sich, dass die erfindungsgemäße Lehre eine keramische Schnittschablone betrifft:
➢ die aus einem Grundkörper 2, mit ein oder mehreren Ausnehmungen 3 zur Durchführung und passgenauen Führung eines Schneideinstruments besteht, wobei die Keramik 3 bis 8 Gew.-% Y₂O₃, vorzugsweise 4 bis 6 Gew.-% Y₂O₃, besonders bevorzugt 4,5 bis 5,5 Gew.-% Y₂O₃, 0 bis 0,5 Gew:-% Al₂O₃, vorzugsweise 0,05 bis 0,4 Gew.-% Al₂O₃, besonders bevorzugt 0,1 bis 0,3 Gew.-% Al₂O₃ und den Rest auf 100 Gew.-% ZrO₂ enthält und wobei bis zu 3 Gew.-% HfO₂, vorzugsweise bis zu 2 Gew.-% HfO₂ im ZrO₂ enthalten sein können und der Monoklinanteil im ZrO₂ vorzugsweise bei weniger als 2 Vol.-%, vorzugsweise bei weniger als 1 Vol.-%. liegt.

Bevorzugt ist eine keramische Schnittschablone, bei der:
➢ die Keramik eine Festigkeit von 900 bis 1600 MPa, vorzugsweise eine Festigkeit von 1000 bis 1500 MPa aufweist;
➢ sich die Korngröße der Keramik in einem Bereich von < 0,5 µm, vorzugsweise in einem Bereich von 0,1 bis 0,3 µm bewegt;
➢ die Ausnehmungen 3 zur Durchführung und passgenauen Führung eines Schneideinstruments schlitzartig sind;
➢ die Ausnehmungen 3 zur Durchführung und passgenauen Führung eines Schneideinstruments Durchgangsbohrungen sind;
➢ die schlitzartigen Ausnehmungen 3 einander gegenüberliegende Führungsflächen 4 aufweisen;
➢ in den Grundkörper 2 zusätzlich ein oder mehrere Durchgangsbohrungen 5 eingebracht sind.

## Patentansprüche

1. Keramische Schnittschablone, **dadurch gekennzeichnet, dass** sie aus einem Grundkörper 2, mit ein oder mehreren Ausnehmungen 3 zur Durchführung und passgenauen Führung eines Schneideinstruments besteht, wobei die Keramik 3 bis 8 Gew.-% Y₂O₃, vorzugsweise 4 bis 6 Gew.-% Y₂O₃, besonders bevorzugt 4,5 bis 5,5 Gew.-% Y₂O₃, 0 bis 0,5 Gew.-% Al₂O₃, vorzugsweise 0,05 bis 0,4 Gew.-% Al₂O₃, besonders bevorzugt 0,1 bis 0,3 Gew.-% Al₂O₃ und den Rest auf 100 Gew.-% ZrO₂ enthält und wobei bis zu 3 Gew.-% HfO₂, vorzugsweise bis zu 2 Gew.-% HfO₂ im ZrO₂ enthalten sein können und der Monoklinanteil im ZrO₂ vorzugsweise bei weniger als 2 Vol.-%, vorzugsweise bei weniger als 1 Vol.-%. liegt.

2. Keramische Schnittschablone gemäß Anspruch 1, **dadurch gekennzeichnet, dass** die Keramik eine Festigkeit von 900 bis 1600 MPa, vorzugsweise eine Festigkeit von 1000 bis 1500 MPa aufweist.

3. Keramische Schnittschablone gemäß Anspruch 1 oder 2, **dadurch gekennzeichnet, dass** sich die Korngröße der Keramik in einem Bereich von < 0,5 µm, vorzugsweise in einem Bereich von 0,1 bis 0,3 µm bewegt.

4. Keramische Schnittschablone gemäß einem der Ansprüche 1 bis 3, **dadurch gekennzeichnet, dass** die Ausnehmungen 3 zur Durchführung und passgenauen Führung eines Schneideinstruments schlitzartig sind.

5. Keramische Schnittschablone gemäß einem der Ansprüche 1 bis 3, **dadurch gekennzeichnet, dass** die Ausnehmungen 3 zur Durchführung und passgenauen Führung eines Schneideinstruments Durchgangsbohrungen sind.

6. Keramische Schnittschablone gemäß Anspruch 4, **dadurch gekennzeichnet, dass** die schlitzartigen Ausnehmungen 3 einander gegenüberliegende Führungsflächen 4 aufweisen.

7. Keramische Schnittschablone gemäß einem oder mehreren der Ansprüche 1 bis 5, **dadurch gekennzeichnet, dass** in den Grundkörper 2 zusätzlich ein oder mehrere Durchgangsbohrungen 5 eingebracht sind.

8. Verwendung der keramischen Schnittschablone nach einem oder mehreren der Ansprüche 1 bis 6 in der Medizintechnik, insbesondere bei Operationen zur Bearbeitung eines Knochens.

9. Verwendung der keramischen Schnittschablone nach einem oder mehreren der Ansprüche 1 bis 6 bei einer Knie-TEP-Implantation.

## Claims

1. A ceramic cutting template, **characterised in that** it consists of a base body 2 having one or more recesses 3 for passing through and guiding, with a precise fit, a cutting instrument, wherein the ceramic material contains 3 to 8 % by weight Y₂O₃, preferably 4 to 6 % by weight Y₂O₃, particularly preferably 4.5 to 5.5 % by weight Y₂O₃, 0 to 0.5 % by weight Al₂O₃, preferably 0.05 to 0.4 % by weight Al₂O₃, particularly preferably 0.1 to 0.3 % by weight Al₂O₃, and the remainder to 100 % by weight ZrO₂, and wherein up to 3 % by weight HfO₂, preferably up to 2 % by weight HfO₂, can be contained in the ZrO₂, and the monoclinic proportion in the ZrO₂ lies preferably at less than 2 % by volume, preferably at less than 1 % by volume.

2. A ceramic cutting template according to claim 1, **characterised in that** the ceramic material has a strength of 900 to 1600 MPa, preferably a strength of 1000 to 1500 MPa.

3. A ceramic cutting template according to claim 1 or 2, **characterised in that** the grain size of the ceramic material lies in a range of < 0.5 µm, preferably in a range of 0.1 to 0.3 µm.

4. A ceramic cutting template according to one of claims 1 to 3, **characterised in that** the recesses 3 for passing through and guiding, with a precise fit, a cutting instrument are slot-like.

5. A ceramic cutting template according to one of claims 1 to 3, **characterised in that** the recesses 3 for passing through and guiding, with a precise fit, a cutting instrument are through-bores.

6. A ceramic cutting template according to claim 4, **characterised in that** the slot-like recesses 3 have opposing guide surfaces 4.

7. A ceramic cutting template according to one or more of claims 1 to 5, **characterised in that** in addition one or more through-bores 5 are introduced into the base body 2.

8. Use of the ceramic cutting template according to one or more of claims 1 to 6 in medical technology, in particular in operations for working on a bone.

9. Use of the ceramic cutting template according to one or more of claims 1 to 6 in a knee-TEP-implantation.

## Revendications

1. Gabarit de coupe en céramique, **caractérisé en ce qu'**il est constitué d'un corps de base 2 doté d'un ou plusieurs évidements 3 pour le passage et le guidage avec ajustage précis d'un instrument de coupe, la céramique contenant de 3 à 8 % en poids de Y₂O₃, de préférence de 4 à 6 % en poids de Y₂O₃, de façon préconisée de 4,5 à 5,5 % en poids de Y₂O₃, de 0 à 0,5 % en poids de Al₂O₃, de préférence de 0,05 à 0,4 % en poids de Al₂O₃, de façon préconisée de 0,1 à 0,3 % en poids de Al₂O₃, et le reste jusqu'à 100 % en poids de ZrO₂, et jusqu'à 3 % en poids de HfO₂, de préférence jusqu'à 2 % en poids de HfO₂. pouvant être contenus dans le ZrO₂, et la proportion monoclinique dans le ZrO₂ étant de préférence inférieure à 2 % en volume, de façon préconisée inférieure à 1 % en volume.

2. Gabarit de coupe en céramique selon la revendication 1, **caractérisé en ce que** la céramique présente une résistance allant de 900 à 1 600 MPa, de préférence une résistance allant de 1 000 à 1 500 MPa.

3. Gabarit de coupe en céramique selon la revendication 1 ou 2, **caractérisé en ce que** la grosseur de grains de la céramique se situe dans une plage < 0,5 µm, de préférence dans une plage allant de 0,1 à 0,3 µm.

4. Gabarit de coupe en céramique selon l'une des revendications 1 à 3, **caractérisé en ce que** les évidements 3 pour le passage et le guidage avec ajustage précis d'un instrument de coupe ont une forme de type fente.

5. Gabarit de coupe en céramique selon l'une des revendications 1 à 3, **caractérisé en ce que** les évidements 3 pour le passage et le guidage avec ajustage précis d'un instrument de coupe sont des trous débouchants.

6. Gabarit de coupe en céramique selon la revendication 4, **caractérisé en ce que** les évidements 3 en forme de fente présentent des surfaces de guidage 4 situées mutuellement en vis-à-vis.

7. Gabarit de coupe en céramique selon une ou plusieurs des revendications 1 à 5, **caractérisé en ce qu'**en plus, un ou plusieurs trous débouchants 5 sont réalisés dans le corps de base 2.

8. Utilisation du gabarit de coupe en céramique selon une ou plusieurs des revendications 1 à 6, dans la technologie médicale, en particulier pour des opérations en vue du traitement d'un os.

9. Utilisation du gabarit de coupe en céramique selon une ou plusieurs des revendications 1 à 6, pour une implantation TEP du genou.
